# EUROPEAN PATENT SPECIFICATION

(11) **EP 1 622 627 B1**
(45) Date of publication and mention of the grant of the patent: **22.05.2013**
(21) Application number: 04730431.6
(22) Date of filing: 30.04.2004
(51) Int. Cl.: C08B 37/00, C08L 5/00, A61K 31/00, A61K 31/716, A61K 36/00, A61K 36/899, A61P 17/00

(54) **PHARMACEUTICAL COMPOSITIONS COMPRISING CEREAL BETA(1-3) BETA(1-4) GLUCAN**
PHARMAZEUTISCHE ZUSAMMENSETZUNGEN ENTHALTEND BETA(1-3) BETA(1-4) GLUCAN AUS GETREIDEN
COMPOSITIONS PHARMACEUTIQUES CONTENANT UN BETA(1-3) BETA(1-4) GLUCANE DE CÉRÉALE

(30) Priority: 02.05.2003 US 467146 P; 10.06.2003 US 477048 P
(43) Date of publication of application: 08.02.2006
(73) Proprietor: Ceapro Inc., Edmonton, AB T5J 4P6 (CA)
(72) Inventor: REDMOND, Mark, J., Edmonton, Alberta T6G 1B3 (CA); FIELDER, David, A., Edmonton, Alberta T6A 2S6 (CA)
(74) Representative: Harding, Charles Thomas
(86) International application number: PCT/CA2004/000662
(87) International publication number: WO 2004/096242

(56) References cited:
- WO-A-00/67626
- WO-A-01/74300
- WO-A-02/43719
- WO-A-03/054077
- US-A- 5 518 710
- US-A- 5 980 918
- ESTRADA A ET AL: "Effect of administration of oat beta-glucan on immune parameters of healthy and immunosuppressed beef steers." CANADIAN JOURNAL OF VETERINARY RESEARCH = REVUE CANADIENNE DE RECHERCHE VETERINAIRE. OCT 1999, vol. 63, no. 4, October 1999 (1999-10), pages 261-268, XP002297977 ISSN: 0830-9000
- ESTRADA ALBERTO ET AL: "Immunomodulatory activities of oat beta-glucan in vitro and in vivo" MICROBIOLOGY AND IMMUNOLOGY, vol. 41, no. 12, 1997, pages 991-998, XP009036004 ISSN: 0385-5600
- CHEUNG NAI-KONG V ET AL: "Orally administered beta-glucans enhance anti-tumor effects of monoclonal antibodies." CANCER IMMUNOLOGY IMMUNOTHERAPY, vol. 51, no. 10, November 2002 (2002-11), pages 557-564, XP002297978 ISSN: 0340-7004
- YUN C-H ET AL: "[beta]-Glucan, extracted from oat, enhances disease resistance against bacterial and parasitic infections" FEMS IMMUNOLOGY AND MEDICAL MICROBIOLOGY 21 FEB 2003 NETHERLANDS, vol. 35, no. 1, 21 February 2003 (2003-02-21), pages 67-75, XP002297979 ISSN: 0928-8244
- BRAATEN J T ET AL: "OAT BETA-GLUCAN REDUCES BLOOD CHOLESTEROL CONCENTRATION IN HYPERCHOLESTEROLEMIC SUBJECTS" EUROPEAN JOURNAL OF CLINICAL NUTRITION, XX, XX, vol. 48, July 1994 (1994-07), pages 465-474, XP001027909

## Description

### TECHNICAL FIELD

The present invention relates generally to cereal β-glucans. More particularly, the invention relates to pharmaceutical compositions comprising a β (1-3) β (1-4) glucan and a botanical extract, or a pharmaceutically active agent.

### BACKGROUND OF THE INVENTION

Gums are either hydrophobic or hydrophilic substances of molecular weight ranging from 10,000 to 50,000,000 Daltons, which in an appropriate solvent produce gels or highly viscous suspensions or solutions at low dry substance content. Gums commonly used in food, medicine, and industrial products include starches, cellulose derivatives, pullulan, agar, aloe, gellan, guar gum, locust bean gum, pectin, algin, carrageenan, xanthan, β-glucan, and gum arabic (see Whistler, R.L. (1993) Industrial Gums: Polysaccharides and their derivatives Eds, Whistler R.L. and BeMiller J.N. (Academic Press) p 2).

Glucans are homopolysaccharides consisting only of glucose. However, since it is possible to link the glucose molecules in different stereochemical configurations, glucans are a diverse group of compounds with differing chemical, physical, and functional properties.

Chemical structures of polysaccharides are of prime importance in determining their properties. This can be appreciated by comparing the properties of some common homoglucans. For example, cellulose, a β (1-4)-D-glucan, is water insoluble and highly crystalline compared to other polysaccharides. Amylose, an α (1-4)-D-glucan, is sparingly soluble in water, crystallizes less well than cellulose, and can form rigid thermo-reversible gels. Dextran, a (1-6)-α-glucan, with a small degree of branching, is extremely water soluble and non-gel forming. (See Dea, I.C.M. in (1993) Industrial Gums: Polysaccharides and their derivatives Eds. Whistler R.L. and BeMiller J.N. (Academic Press) p 21).

Oat β (1-3) β (1-4) glucan is classified as a viscous gum, (see Wood, P.J. (1993) Oat Bran Ed P.J. Wood (American Association of Cereal Chemists, Inc. St. Paul, MN)). Cereal β (1-3) β (1-4)-glucans are structural polysaccharides present in the cell walls of cereals, such as barley and oat, among others.

Oat β (1-3) β (1-4) glucan is recognised by the U.S. FDA as an agent that may aid in the prevention of heart disease. In 1997, the FDA allowed oat products to make a health claim. It is important to note that no other β-glucan source, yeast, fungal, bacterial, or cereal is recognised as having these effects. Oat β (1-3) β (1-4)-glucans are therefore distinct.

Unmodified oat β (1-3) β (1-4) glucan forms highly viscous solutions in water at concentrations >0.75%. At concentrations >1.2% the solutions have the consistency of a thick hydrogel.

Glucans of significantly different molecular structure and with different physical and chemical properties compared to oat are found in yeast, fungi, and certain bacteria and genetically engineered bacteria. For example, gellan, polymeric (1-3) β-D-glucopyranosyl [β (1-3)-glucan] produced in *Alcaligenes faecalis* is found in Curdlan (Takeda Chemical Ind. Ltd.), β (1-3) α (1-6) glucopyranoside produced in *Aureobasidium pullulans* is found in pullulan, and β (1-3) β (1-6) glucopyranoside is found in yeast.

The molecular weight of the glucans varies with source. Table 1 shows the average molecular weight of typical gums.

**Table 1: Typical Molecular Weight Range of Common Gums**

| **GUM** | **AVERAGE MOLECULAR WEIGHT** |
|---|---|
| Oat β (1-3) β (1-4) glucan | 500,000 - 1,000,000 |
| Pullulan | 50,000 - 100,000 |
| Curdlan | ∼500,000 |
| Methyl cellulose | 10,000 - 200,000 |
| Carrageenan | 4,500,000 |
| Xanthan | 15,000,000 - 50,000,000 |
| Sodium alginate | 10,000 - 18,000,000 |

The viscosity of a 1% solution of different polysaccharide gum solutions varies with origin and chemical nature. Table 2 shows the viscosity of 1% solutions of typical gums.

**TABLE 2: Typical Viscosity Ranges of 1% Solutions of Common Gums, Measured at 25°C**

| **GUMS** | **1% SOLUTION VISCOSITY, cP** |
|---|---|
| Oat β (1-3) β (1-4) glucan | 500-1500 |
| Pullulan | 2 |
| Gum Arabic | 1-5 |
| Methyl cellulose | 200 |
| Tamarind gum | 100-200 |
| Guar gum | 2,000-3,000 |
| Locust bean gum | 2,000-3,000 |
| Xanthan | 2,000-3,000 |
| Sodium alginate | 200-700 |

The solubility properties of glucans differ according to their source. For example, cereal β (1-3) β (1-4) glucans are normally soluble in aqueous solvents, whereas yeast (*Saccharomyces cerevisiae)* β (1-3) β (1-6)-*glucans* are insoluble in aqueous solvents. Soluble-glucans are desirable. Yeast β-glucan has been solubilized by the addition of phosphate groups (see Williams et al. Immunopharmacol. 22: 139-156 (1991). Jamas et al. (U.S. Patent No. 5,622,939) describes methods to extract soluble β (1-3) β (1-6) glucan from *Saccharomyces cerevisiae.* The method described is complex involving acid hydrolysis, base hydrolysis and the extensive use of centrifugation and ultrafiltration. No details are provided as to the stability of the solubilized yeast β (1-3) β (1-6) glucan.

A number of prior art references disclose methods of preparing-glucans and liquid-glucan compositions from cereals. Among these prior art references are the following:

Beer, et al., Extraction of Oat Gum from Oat Bran: Effects of Process on Yield, Molecular Weight Distribution, Viscosity and (1-3) (1-4) beta-D-Glucan Content of the Gum, Cereal Chemistry 73(1): 58-62 (1996). This reference describes the use of alcohols in an amount equal to or greater than 50% (v/v) to achieve precipitation. The purity of the recovered glucans was reported to be between 22 and 63%.

Wood et al., Large Scale Preparation and Properties of Oat Fractions Enriched in (1-3)(1-4) beta-D-Glucan, Cereal Chemistry 66(2): 97-103 (1989). This reference describes the use of alcohols in an amount equal to or greater than 50% (v/v) to achieve precipitation of glucans.

U.S. Patent No. 6,323,338 discloses a method of isolating oat β-glucan as an enriched skin from an extract of oat bran. The disclosed method does not utilize low concentrations of short-chain alcohols for the precipitation of the glucan.

Redmond (U.S. Patent No. 6,284,886) discloses compositions of cereal β (1-3) β (1-4) glucans, and methods of producing these compositions. The disclosed compositions meet the strict requirements of the cosmetics industry, in terms of their viscosity, shear strength, and moisture-enhancing properties. No method for the extraction or purification of β (1-3) β (1-4) glucan is described.

### SUMMARY OF THE INVENTION

The present invention relates generally to cereal β-glucans. More particularly, the invention relates to pharmaceutical compositions comprising a β (1-3) β (1-4) glucan and a botanical extract, or a pharmaceutically active agent.

The present invention can be summarised using the following numbered paragraphs:-
1. A pharmaceutical composition comprising:
   a β (1-3) β (1-4) glucan, and
   a botanical extract, or a pharmaceutically active agent,
   wherein the β (1-3) β (1-4) glucan is produced by a method comprising:
      (i) extracting a milled cereal grain or a milled part of the cereal grain with an alkaline solution to produce an extract containing at least about 0.4 weight percent β (1-3) β (1-4) glucan;
      (ii) removing insoluble material, and removing particulate material having a particle size of greater than about 0.2 µm from said extract to produce a purified extract;
      (iii) adding from about 10% to about 25% (vol/vol) of a C₁-C₄ alcohol to the purified extract to precipitate the β (1-3) β (1-4) glucan, and
      (iv) isolating the β(1-3) β(1-4) glucan.
2. The pharmaceutical composition according to paragraph 1, wherein the composition comprises the botanical extract, and wherein the botanical extract is an extract of Guarana, *Ginkgo biloba,* Kola nut, Goldenseal, Golo Kola, *Schizandra,* Elderberry, St. John's Wort, Valerian and *Ephedra,* black tea, white tea, java tea, garlic oil, fiber, green tea, lemon oil, mace, licorice, onion oil, orange oil, rosemary, milk thistle, *Echinacea,* Siberian ginseng or *Panax ginseng,* lemon balm, *Kava kava,* matte, bilberry, soy, grapefruit, seaweed, hawthorn, lime blossom, sage, clove, basil, curcumin, taurine, wild oat herb, oat grain, dandelion, gentian, aloe vera, hops, cinnamon, peppermint, grape, chamomile, fennel, marshmallow, ginger, slippery elm, cardamon, coriander, anise, thyme, rehmannia, eucalyptus, menthol, schisandra, withania, cowslip, lycium, or passion flower.
3. The pharmaceutical composition according to paragraph 2, wherein the botanical extract is an extract of oat grain.
4. The pharmaceutical composition of paragraph 3, wherein the botanical extract comprises avenanthramide.
5. The pharmaceutical composition according to paragraph 1, wherein the composition comprises the pharmaceutically active agent, and wherein the pharmaceutically active agent is selected from the group consisting of beta-sitosterol, caffeine, cafestol, D-limonene, kabweol, nomilin, oltipraz, sulphoraphane, tangeretin, folic acid, and menthol.
6. The pharmaceutical composition according to paragraph 1, wherein the composition comprises the pharmaceutically active agent, and wherein the pharmaceutically active agent is selected from the group consisting of an antihistamine, a decongestant, a corticosteroid, a non-steroidal anti-inflammatory drug, a bronchodilator, a vasodilator, such as nitroglycerin, and a local anaesthetic.
7. The pharmaceutical composition of paragraph 6, wherein the vasodilator is nitroglycerin.
8. The pharmaceutical composition according to paragraph 1, wherein the β (1-3) β (1-4) glucan is derived from a cereal grain or a part of the cereal grain.
9. The pharmaceutical composition according to paragraph 8, wherein the cereal is selected from the group consisting of a cultivar of barley, a cultivar of oat, a cultivar of wheat, a cultivar of rye, a cultivar of sorghum, a cultivar of millet, a cultivar of corn, and a mixture thereof.
10. The pharmaceutical composition according to paragraph 1, wherein the β (1-3) β (1-4) glucan is a β (1-3) β (1-4) glucan composition having a purity of at least about 75%, and containing less than 10% ash impurities, less than 10% protein impurities, and less than 5% lipid impurities.
11. The pharmaceutical composition according to paragraph 10, wherein the β (1-3) β (1-4) glucan composition has a purity of at least about 92%, and contains less than 3.5% ash impurities, less than 3.5 % protein impurities, and less than 1% lipid impurities.
12. The pharmaceutical composition according to paragraph 10, wherein the cereal β-glucan composition has a clarity value of from about 5 to about 100 NTU.
13. The pharmaceutical composition according to paragraph 1, wherein in said step of adding (step iii) in said method, about 10% to about 20% (vol/vol) of an alcohol selected from the group consisting of methanol, ethanol and isopropanol, is used to precipitate the β (1-3) β (1-4) glucan composition from said purified extract.
14. The pharmaceutical composition according to paragraph 13, wherein about 10% to about 20% (vol/vol) of ethanol is used to precipitate the β (1-3) β (1-4) glucan composition from said purified extract.
15. The pharmaceutical composition according to paragraph 1, wherein said step of removing particulate material in said method comprises:
   one, or more than one step of adding a flocculant, a coagulant or both the flocculant and the coagulant to said extract to coagulate particulate material having a particle size of greater than about 0.2 µm, and removing coagulated material from said extract,
   digesting starch material in said extract, and
   filtering out particulate material having a particle size of greater than about 0.2 µm from said extract to produce the purified extract.
16. The pharmaceutical composition according to paragraph 15, wherein in said step of digesting in said method, said starch material is digested with an enzyme.
17. The pharmaceutical composition according to paragraph 16, wherein prior to digesting said starch material, said alkaline solution is neutralized.
18. The pharmaceutical composition according to paragraph 17, wherein following the digestion of said starch material in said method, said enzyme is inactivated.
19. The pharmaceutical composition according to paragraph 18, wherein said enzyme is inactivated by acidifying the neutralized solution.
20. The pharmaceutical composition according to paragraph 16, wherein said enzyme is an amylase.
21. The pharmaceutical composition according to paragraph 20, wherein said amylase does not require a calcium cofactor.
22. The pharmaceutical composition according to paragraph 1, wherein the cereal is selected from the group consisting of a cultivar of barley, a cultivar of oat, a cultivar of wheat, a cultivar of rye, a cultivar of sorghum, a cultivar of millet, a cultivar of corn, and a mixture thereof.
23. The pharmaceutical composition according to paragraph 1, wherein the alkaline solution has a value of pH of between 9 to 10.
24. The pharmaceutical composition according to paragraph 1, wherein the pH of the alkaline solution used in said method is from about 9.25 to about 9.75.
25. The pharmaceutical composition according to paragraph 1, wherein said step of adding (step iii) in said method is conducted at a temperature of from about 1°C to about 10°C.
26. The pharmaceutical composition according to paragraph 1, wherein said method further comprises one, or more than one step of dissolving the isolated β (1-3) β (1-4) glucan in an aqueous solution, precipitating the β (1-3) β (1-4) glucan by adding about 10% to about 25% (vol/vol) of the C₁-C₄ alcohol to the aqueous solution, and isolating the β (1-3) β (1-4) glucan.
27. The pharmaceutical composition according to paragraph 23 or 24, wherein the milled cereal grain or the milled part of the cereal grain is extracted with the alkaline solution for a period of time of about 15 to about 45 minutes to produce the extract containing at least about 0.4 weight percent β (1-3) β (1-4) glucan.
28. The pharmaceutical composition according to paragraph 15, wherein the step of filtering out material having a particle size of greater than 0.2 µm from said extract is conducted using a filter coated with a pre-coat of a filter aid having a porosity of 0.2 µm.

In a first aspect, the present description provides a pharmaceutical composition comprising:
an effective amount of a β (1-3) β (1-4) glucan, and
an effective amount of a botanical extract, or a pharmaceutically active agent,

In one example, the composition of the first aspect of the present description comprises:
an effective amount of a β (1-3) β (1-4) glucan, and
an effective amount of a botanical extract, wherein the botanical extract is an extract of Guarana, *Ginkgo biloba,* Kola nut, Cloldenseal, Golo Kola, *Schizandra,* Elderberry, St. John's Wort, Valerian and *Ephedra,* black tea, white tea, java tea, garlic oil, fiber, green tea, lemon oil, mace, licorice, onion oil, orange oil, rosemary, milk thistle, *Echinacea,* Siberian ginseng or *Panax ginseng,* lemon balm, *Kava kava,* matte, bilberry, soy, grapefruit, seaweed, hawthorn, lime blossom, sage, clove, basil, curcumin, taurine, wild oat herb, oat grain, dandelion, gentian, aloe vera, hops, cinnamon, peppermint, grape, chamomile, fennel, marshmallow, ginger, slippery elm, cardamon, coriander, anise, thyme, rehmannia, eucalyptus, menthol, schisandra, withania, cowslip, lycium, or passion flower, or
an effective amount of a pharmaceutically active agent selected from the group consisting of beta-sitosterol, caffeine, cafestol, D-limonene, kabweol, nomilin, oltipraz, sulphoraphane, tangeretin, folic acid, and menthol.

In another example of the composition of the first aspect of the present description, the botanical extract is an extract of oat grain, which preferably comprises avenanthramide.

In another example, the composition of the first aspect of the present description comprises:
an effective amount of a β (1-3) β (1-4) glucan, and
an effective amount of a pharmaceutically active agent selected from the group consisting of an antihistamine, a decongestant, a corticosteroid, a non-steroidal anti-inflammatory drug, a bronchodilator, a vasodilator, such as nitroglycerin, or a local anaesthetic.

The β (1-3) β (1-4) glucan of the above-defined pharmaceutical compositions may be derived from a cereal grain or a part of the cereal grain. In an example, the cereal is selected from the group consisting of a cultivar of barley, a cultivar of oat, a cultivar of wheat, a cultivar of rye, a cultivar of sorghum, a cultivar of millet, a cultivar of corn, and a mixture thereof.

The β (1-3) β (1-4) glucan used in the pharmaceutical compositions of the present description may be a β (1-3) β (1-4) glucan composition having a purity of at least about 75%, and containing less than 10% ash impurities, less than 10% protein impurities, and less than 5% lipid impurities. More particularly, the present description relates to a pharmaceutical composition comprising a β (1-3) β (1-4) glucan composition having a purity of at least about 92%, and containing less than 3.5% ash impurities, less than 3.5 % protein impurities, and less than 1% lipid impurities. The cereal β-glucan composition can also have a clarity value of from about 5 to about 100 NTU.

The β (1-3) β (1-4) glucan used in the pharmaceutical compositions of the present description may be formed by a method of isolating a β (1-3) β (1-4) glucan from a milled cereal grain or a milled part of the cereal grain, comprising:
(i) extracting the milled cereal grain or the milled part of the cereal grain with an alkaline solution to produce an extract containing at least about 0.4 weight percent β (1-3) β (1-4) glucan;
(ii) removing insoluble material, and removing particulate material having a particle size of greater than about 0.2 µm from the extract to produce a purified extract;
(iii) adding from about 10% to about 25% (w/w) of a C₁-C₄ alcohol to the purified extract to precipitate the β (1-3) β (1-4) glucan, and
(iv) isolating the β(1-3) β(1-4) glucan.

In an example of the above-defined method, about 10% to about 20% (w/w) of an alcohol selected from the group consisting of methanol, ethanol and isopropanol is used to precipitate the β (1-3) β (1-4) glucan from the filtrate. Preferably, about 10% to about 20% (w/w) of ethanol is used to precipitate the β (1-3) β (1-4) glucan.

In an example of the methods described above, the step of removing particulate material comprises:
one, or more than one step of adding a flocculant, a coagulant or both a flocculant and a coagulant to the extract to coagulate particulate material having a particle size of greater than about 0.2 µm, and removing coagulated material from the extract;
digesting starch material in the extract, and
filtering out particulate material having a particle size of greater than about 0.2 µm from the extract to produce a purified extract.

In an example of the just described method, the starch material is digested with an enzyme, such as an amylase. More particularly, the enzyme is digested with an amylase that does not require a calcium cofactor. In another example, the alkaline extract is neutralized before the starch material is digested. In a further example, the enzyme is inactivated following the digestion of the starch material, by, for example, acidifying the alkaline extract containing the digested starch material.

The cereal used in the methods described above may be selected from the group consisting of a cultivar of barley, a cultivar of oat, a cultivar of wheat, a cultivar of rye, a cultivar of sorghum, a cultivar of millet, a cultivar of corn, and a mixture thereof.

In another example, the cereal grain or the part of the cereal grain extracted in step (i) is in the form of a coarsely-milled flour or a finely-milled flour.

In other examples of the above-described methods, the pH value of the alkaline solution is from about 9.00 to about 10.00, from about 9.25 to about 9.75, or from about 9.30 to about 9.50. In another example, the step of extracting (step i) is carried out over a period of about 15 to about 45 minutes.

In a further example of the above-defined methods, the precipitation step is conducted at a temperature of from about 1°C to about 10°C, or from about 1°C to about 5°C. In an even further example, the alcohol used to conduct the precipitation step is cooled to a temperature of at least about -20°C before being added to the β(1-3) β(1-4) glucan solution.

The methods defined above may further comprise one, or more than one step of dissolving the isolated β (1-3) β (1-4) glucan from step (iv) in an aqueous solution, precipitating the β (1-3) β (1-4) glucan by adding about 10% to about 25% (w/w) of the C₁-C₄ alcohol to the aqueous solution, and isolating the β (1-3) β (1-4) glucan.

In a further example, the β (1-3) β (1-4) glucan used in the pharmaceutical compositions of the present description may be formed by a method of isolating a β (1-3) β (1-4) glucan from a milled cereal grain or a milled part of the cereal grain, comprising:
(i) extracting the milled cereal grain or the milled part of the cereal grain with an alkaline solution to produce an extract comprising at least about 0.4 weight percent β (1-3) β (1-4) glucan;
(ii) removing insoluble material, and removing particulate material having a particle size of greater than about 0.2 µm from the extract to produce a purified extract, wherein the step of removing particulate material comprises:
   one, or more than one step of adding a flocculant, a coagulant or both a flocculant and a coagulant to the extract to coagulate particulate material having a particle size of greater than about 0.2 µm, and removing coagulated material from the extract;
   enzymatically digesting starch material in the extract, and
   filtering out particulate material having a particle size of greater than about 0.2 µm from the extract to produce the purified extract;
(iii) adding about 10% to about 25% (w/w) of a C₁-C₄ alcohol to the purified extract to precipitate the β(1-3) β(1-4) glucan, and
(iv) isolating the β(1-3) β(1-4) glucan.

The purification method of the present description differs from the method disclosed in U.S. Patent No. 6,323,338 in that fine particulate matter is removed, as well as a large proportion of protein (∼90%) present in the original cereal grain.

The purification method of the present description allows the use of concentrations of alcohol of less than 50% (w/w), for example, 10-25% aqueous alcoholic solutions to precipitate cereal β-glucan. The capability of using such concentrations of alcohol is surprising in view of prior art purification procedures, which have used 50% ethanol solutions to precipitate cereal β-glucan (see, for example, Wood et al. Large Scale Preparation and Properties of Oat Fractions Enriched in β (1-3) β (1-4) D-glucan Cereal Chem. 66 97-103 (1989)). It is believed that the removal of the particulate matter and most of the protein material, according to the method of the present description, reduces the amount of alcohol needed to precipitate the cereal β-glucan from solution.

The use of 10-25% aqueous alcoholic solutions to precipitate the cereal β-glucan is advantageous in that severe dehydration of the cereal β-glucan is avoided, resulting in a cereal β-glucan precipitate that can be easily suspended in water. Furthermore, the use of these relatively lower alcohol concentrations allows the starting cereal grain to be processed in a standard manufacturing plant without the need for explosion proof environmental systems. For example, use of 20% aqueous alcoholic solutions at a final temperature of 7-10°C produces a vapor pressure lower than the Lower Explosion Limit (LEL) of ethanol. Furthermore, the efficiency of the extraction step and the production of intermediate solutions containing cereal β-glucan at a concentration of greater than 0.4% permits processing using relatively small process volumes.

### DESCRIPTION OF PREFERRED EMBODIMENT

The present invention relates generally to cereal β-glucans. More particularly, the invention relates to pharmaceutical compositions comprising a β (1-3) β (1-4) glucan and a botanical extract, or a pharmaceutically active agent, wherein the β(1-3) β (1-4) glucan is produced by the method described in claim 1.

The practice of the present invention will employ, unless otherwise indicated, conventional methods of chemistry, cereal chemistry, and biochemistry, within the skill of the art. Such techniques are explained fully in the literature. See for example, Industrial Gums: Polysaccharides and their derivatives Eds. Whistler R.L. and BeMiller J.N. (Academic Press), Oats: Chemistry and Technology ed. Webster F.H. (American Association of Cereal Chemists, St. Paul, MN).

As used in this specification and the appended claims, the singular forms "a", "an" and "the" include plural references unless the content clearly indicates otherwise.

### Definitions

In describing the present invention, the following terms will be employed, and are intended to be defined as indicated below.

By "cereal" is meant any of several grains such as, but not limited to, cultivars of barley, oat, wheat, rye, sorghum, millet, and corn.

By "glycan" is meant a polymer of monosaccharides linked together by glycosidic bonds.

By "glucan" is meant a homopolysaccharide consisting only of glucose.

By "cereal β-glucan" is meant a glucan with a β (1-3)-linked glucopyranosyl backbone, or a β (1-4)-linked glucopyranosyl backbone, or a mixed β (1-3) β (1-4)-linked glucopyranosyl backbone, which is derived from a cereal source.

By "β (1-3) β (1-4) glucan" is meant a cereal β-glucan.

By "gum" is meant a plant or microbial polysaccharide or their derivatives, which are dispersible in either cold or hot water to produce viscous mixtures or solutions. Gums may be classified by origin, and include: exudate gums, seaweed gums, seed gums, starch and cellulose derivatives, and microbial gums.

By "compound of interest" is meant any pharmaceutical, medicinal, botanical or therapetic material mixed with a β (1-3) β (1-4) glucan to produce a composition.

By "flocculant" and "coagulant" are meant molecules that can coalesce with suspended solids (fines) to form larger denser particles that can be separated by centrifugation. In particular examples, coagulants are molecules that can bring together suspended particles that are less than 1 µm in size, and flocculants are molecules that can bring together suspended particles that are greater than 1 µm in size.

By "insoluble material" is meant material that is not soluble under the initial alkaline extraction conditions of the method of the description. Non-limiting examples of such material include fibre, hemicellulose and lignins.

By "particulate material" is meant a solid or colloidal material having a particle size of greater than about 0.2 µm.

By "a milled cereal grain" or "a milled part of the cereal grain" is meant a cereal grain or part of the cereal grain, which has been ground, abraded or chopped into a meal or flour. In a particular example, the milled part of the cereal grain is bran that has been abraded from the cereal grain, and optionally further ground and purified by, for example, air classification or sieving to provide a specific particle profile.

By "effective amount" is meant the amount of the one, or more than one compound of interest necessary to achieve a desired effect, such as a physiological effect, or a stimulatory effect.

By "sequestered" is meant the incorporation, entrapment, or solubilization of hydrophilic compounds, or hydrophobic compounds, for example, small molecular weight hydrophobic compounds, such as essential oils, pharmaceutical, medicinal, and therapeutic agents.

The compositions of the present invention can be formed by mixing an aqueous solution comprising about 0.01 wt. % to about 20 wt. %, about 0.01 wt. % to about 1.2% wt. %, about 0.1 wt. % to about 1.1 wt. %, or about 0.5 wt. % to about 1 wt. % of the β (1-3) β (1-4) glucan with one, or more than one compound of interest, such as a botanical extract, or a pharmaceutically active agent. The one, or more than one compound of interest may be present in an amount of from about 0.01 wt. % to about 40 wt. %, from about 0.01 wt. % to about 25 wt. %, from about 0.01 wt% to about 4 wt. %, from about 0.1 wt. % to about 1.4 wt. %, or from about 0.5 wt. % to about 1.2 wt. %. It is preferred that the resulting compositions be left undisturbed after being mixed for a period of time sufficient to allow the formation of a homogeneous composition in the form of a suspension, emulsion or gel. In many cases, the amount of time required to obtain a homogeneous composition is from about eight to about 16 hours.

It is to be appreciated, however, that shorter or longer periods of time may be required, depending on the quantity and purity of the β (1-3) β (1-4) glucan used, as well as on the quantity and nature of each of the one, or more than one compound of interest. Compositions of the present invention, which are in the form of a gel may be converted into a more fluid state by gentle agitation.

Without wishing to be bound by theory, the formation of the homogeneous suspension, emulsion or gel is believed to be caused by the one, or more than one compound of interest being sequestered or encapsulated within the β (1-3) β (1-4) glucan, and by the subsequent formation of hydrogen bonds between molecules of the one, or more than one compound of interest and the β (1-3) β (1-4) glucan. Another possibility is that the β (1-3) β (1-4) glucan acts as a surfactact or emulsifying agent by reducing the interfacial tension at the boundaries between the one, or more than one compound of interest and the aqueous phase within which the β (1-3) β (1-4) glucan is dispersed, and, consequently, effectively solubilizes the one, or more than one compound within the aqueous phase.

Examples of the botanical extract that may be used in the pharmaceutical compositions according to the present invention include, without limitation, extracts of Guarana, *Ginkgo biloba,* Kola nut, Goldenseal, Golo Kola, *Schizandra,* Elderberry, St. John's Wort, Valerian and *Ephedra,* black tea, white tea, java tea, garlic oil, fiber, green tea, lemon oil, mace, licorice, onion oil, orange oil, rosemary, milk thistle, *Echinacea,* Siberian ginseng or *Panax ginseng,* lemon balm, *Kava kava,* matte, bilberry, soy, grapefruit, seaweed, hawthorn, lime blossom, sage, clove, basil, curcumin, taurine, wild oat herb, oat grain, dandelion, gentian, aloe vera, hops, cinnamon, peppermint, grape, chamomile, fennel, marshmallow, ginger, slippery elm, cardamon, coriander, anise, thyme, rehmannia, eucalyptus, menthol, schisandra, withania, cowslip, lycium, and passion flower.

In a particular example, the botanical extract is an extract of an oat grain. More particularly, the botanical extract is an oat grain extract, which contains avenanthramide.

As an example of a pharmaceutically active agent there is mentioned an antihistamine, a decongestant, a corticosteroid, a non-steroidal anti-inflammatory drug, a bronchodilator, a vasodilator, or a local anaesthetic.

Other examples of the pharmaceutically active agent botanical extract that may be used in the pharmaceutical compositions of the present invention include, without limitation, beta-sitosterol, caffeine, cafestol, D-limonene, kabweol, nomilin, oltipraz, sulphoraphane, tangeretin, folic acid, and menthol.

Cereal beta glucans suitable for use in making the compositions of the present invention are available in powdered form from several commercial suppliers, such as Sigma Chemical Co. (St. Louis, MO) and Ceapro Inc. (Edmonton, AB, Canada). Solutions of beta glucan can be prepared in the manner described in U.S. Patent No. 6,284,886.

The beta glucan solutions used in preparing the compositions of the present invention may also be prepared from a beta glucan composition having a purity of from about 65% to about 100%, from about 75% to about 100%, or from about 85% to about 100%. In particular, beta glucan solutions used in preparing the compositions of the present invention generally contain less than 20%, more particularly less than 15%, even more particularly less than 10%, most particularly less than 5% of impurities, such as protein, lipid, carbohydrate, and particulate impurities.

The β (1-3) β (1-4) glucan used in the pharmaceutical compositions of the present invention may be formed by a method of isolating a β (1-3) β (1-4) glucan from a milled cereal grain or a milled part of the cereal grain, comprising:
(i) extracting the milled cereal grain or the milled part of the cereal grain with an alkaline solution to produce an extract containing at least about 0.4 weight percent β (1-3) β (1-4) glucan;
(ii) removing insoluble material, and removing particulate material having a particle size of greater than about 0.2 µm from the extract to produce a purified extract;
(iii) adding from about 10% to about 25% (w/w) of a C₁-C₄ alcohol to the purified extract to precipitate the β (1-3) β (1-4) glucan, and
(iv) isolating the β(1-3) β(1-4) glucan.

Cereal β-glucan can be isolated according to the purification method of the present invention from a milled whole cereal grain or a milled part of the cereal grain, such as the milled bran of the cereal grain. Preferably, the bran of the cereal grain is used. The cereal grain, or a part thereof that is extracted may be in the form of a coarsely milled meal or finely milled flour. The cereals that can be used in the present invention include, without limitation, any one of the cultivars of barley, oat, wheat, rye, corn, sorghum, and millet.

In the first step of the purification method of the present invention, the milled grain or the milled part of the grain is slurried with reverse osmosis (RO) purified or deionized (DI) water to a final solids concentration of about 4 to about 10%, or about 6 to about 8%.

The pH value of the water used in the first step of the purification method can be from about 9.00 to about 10.00, more particularly, from about 9.25 to about 9.75, or from about 9.30 to about 9.50, and can be adjusted using an inorganic base, such as sodium hydroxide or potassium hydroxide. In one example, potassium hydroxide is used at a concentration from about 28 mM to about 35 mM. The use of a solution having a value of pH of between 9-10 generally reduces the amount of non-glucan polysaccharides and protein that is extracted during the first step, and, therefore, provides selective extraction of high molecule weight cereal β glucan molecules.

The extraction of the cereal β-glucan can be carried out over a 15 to 45 minute period, or over a 15-30 minute period. It is to be appreciated, however, that longer or shorter periods of extraction may be used depending on the type of cereal β-glucan used.

In the second step of the purification process, any insoluble material that cannot be extracted, for example hemicelluloses or lignins, is removed. Examples of methods that can be used to separate the insoluble material include, without limitation, centrifugation, preferably with a decanter centrifuge, and vibrating screening.

Any fine particulate material including some protein-based material is also removed from the alkaline solution in the second step of the method of the present invention. This material can be removed by adding an external flocculant or coagulant, or both. The flocculants or coagulants that can be used in the second step can have either a net positive, negative, or neutral charge. The coagulation step may be repeated one, or more than one time.

Examples of flocculants that can be used include, without limitation synthetic flocculants, such as polyacrylamides, quaternary acrylate salts and natural flocculant macromolecules such as chitosan, a natural polymer derived from chitin. Particular examples of flocculants include Tramfloc^{®} (Tramfloc Inc.), the cationic flocculant SURFLOC^{®} 34030 (Jes-Chem Ltd.), polyacrylamide (PAM) flocculants such as an Aquamark^{®} AQ 600 Series flocculant; or a SuperFloc^{®} C-500 Series flocculant (QEMI Inc.).

Examples of coagulants that can be used in the method of the present invention include, without limitation, inorganic electrolytes, such as alum, lime, ferric chloride, and ferrous sulfate, organic polymers, synthetic polyelectrolytes with anionic or cationic functional groups, and polyacrylamides.

The flocculants, coagulants, or a mixture thereof, may be used at a concentration of from about 0.09% to about 0.20% (w/vol), or from about 0.10% to about 0.13% (w/vol).

The alkaline solution may be incubated with the flocculant or coagulant for about 10 to about 40 minutes, or from about 10 to about 20 minutes at a temperature of from about 20 to about 40°C, or from about 20 to about 30°C. It is to be appreciated, however, that longer or shorter periods of time can be used to effect coagulation of the particulate material.

If negatively charged materials are to be removed from the solution containing the cereal β-glucan, then it is preferred that the flocculant or coagulant be positively charged. If positively charged materials are to be removed from the solution, then a negatively-charged flocculant or coagulant is preferred.

Without wishing to be bound by theory, the flocculants and coagulants that can be used in the method of the present invention function by forming large, dense aggregates with fine particulate matter, which can be easily separated from the aqueous solution containing the cereal β-glucan.

The coagulated material may be removed by centrifugation, using, for example, a disk-stack centrifuge. Other physical separation methods known to those of skill in the art can also be used to effect the separation of the coagulated material.

In the second step, any starch or related material that is present may be digested using an enzyme, such as, but not limited to an amylase. The enzyme may be used at a concentration of from about 0.05% to about 0.20% (vol/vol), from about 0.09% to about 0.15% (vol/vol), or from about 0.09% to about 0.11% (vol/vol). If an amylase is used, it is preferred that the alkaline solution be brought to an approximately neutral value of pH (i.e. -pH 7) before adding the amylase. In an example, the solution containing the amylase is heated to a temperature of from about 50°C to about 100°C, or from about 70°C to about 90°C for about 20 to about 30 minutes to gelatinize the starch. The amylase will hydrolyse the starch and any related material. Generally, the amylase that is chosen to break down the starch material should be functional and stable within the temperature ranges indicated above. It is particularly preferred that the amylase not require a calcium co-factor to digest the starch material. Examples of such an amylase, include, without limitation, Termamyl^{®} LC (Novozymes A/S), and Spezyme^{®} FRED (Genencor International Inc.).

The completion of the hydrolysis reaction is determined when a sample withdrawn from the solution no longer produces a positive iodine test. At this point, the enzyme may be inactivated, by, for example, reducing the pH to a value of about 3.5 to about 4.0. The pH of the solution can be reduced using strong inorganic acids, such as hydrochloric acid or weak organic acids, such as malic acid or citric acid. It is preferred, however, that a strong inorganic acid, such as hydrochloric acid be used. In addition, it is preferred that the temperature of the solution be raised to between 85-90°C to denature protein present in solution.

The resulting acidified solution can then be filtered to remove any particulates and microbiological contaminants, through a filter pad that preferably has a cutoff point of about 20 µm. This filter may be coated with a pre-coat of a filter aid having a thickness of about 2 to about 5 mm, such as Celite^{®} C65 (World Minerals), which has a nominal porosity of about 0.2 µm. An equivalent weight of a filter-aid, for example, an acid-washed pharmaceutical grade filter-aid, such as Celite^{®} C300 (World Minerals), may also be added as a body feed to the acidified solution prior to conducting the filtration step.

The filtration can be conducted using any one of a number of filtration devices. One particular example of a filtration device that can be used is a filter-press. In the case where the particle size of the material contained in the extract is less than 0.5 microns, then ceramic microfiltration and ultrafiltration can alternatively be used to filter the acidified solution.

In the third step of the purification method, the cereal β-glucan is precipitated from solution by adding a C₁-C₄ alcohol. The alcohol used to precipitate out the cereal β-glucan may be selected from the group consisting of methanol, ethanol, and isopropanol. If the cereal β-glucan isolated according to the procedure of the present invention is to be used in the preparation of a pharmaceutical, or an edible product, then it is preferred that ethanol or isopropanol, more preferably, ethanol, be used.

As the concentration of the alcohol in the solution is increased, the cereal β-glucan is precipitated out as a fine colloidal suspension. The total amount of alcohol that is required to carry out the precipitation step may depend on the concentration of cereal β-glucan in solution. The alcohol is added to a final concentration of about 10% to about 25% by volume, preferably from about 15% to about 17% by volume. The present invention, therefore, avoids the use of high concentrations of alcohol (i.e. concentrations of greater than 50% by volume), which can cause severe dehydration of the cereal β-glucan and result in the need for homogenizers to disperse the cereal β-glucan.

It is preferred that the precipitation step be conducted at a low temperature, such as from about 1°C to about 10°C, preferably from about 1°C to about 5°C. In addition, it is preferred that the alcohol used in the precipitation step be cooled to a temperature of at least about -20°C before adding it to the β(1-3) β(1-4) glucan solution.

The final isolated cereal β-glucan material is a microdispersion or a nanodispersion, which is free of large particulates, and does not require additional filtration. Aqueous solutions of the cereal β-glucan isolated according to the present invention remain homogeneous after more than a year of being prepared.

Centrifugation using, for example, a disk-stack centrifuge, or a hydrocyclone can be used to isolate the suspended cereal β-glucan. If desired, the isolated β-glucan can be re-dissolved in an aqueous solution and re-precipitated with the C₁-C₄ alcohol to increase the purity of the β-glucan. The isolated solid can then be dried to a powder using, for example, vacuum drying, spray drying or drum drying. The preferred method of drying is vacuum drying, which produces a coarsely granular solid that can be further milled to a desired particle size, for example by hammer, pin or jet-milling. Vacuum drying, however, requires less heat, and can produce a relatively purer cereal β-glucan since Maillard and other by-products are minimized.

To prevent gellation of the cereal β-glucan at each of the steps of the purification method of the present description, it is preferred that the addition of salts be minimized throughout the process. For example, it is preferred that reverse osmosis (RO) purified or deionized (DI) water be used, as well as an amylase not requiring a calcium cofactor, such as Termamyl^{®} LC (Novozymes A/S).

Without wishing to be bound by theory, one way in which gellation of solutions of cereal β-glucan can take place is by cross-linking of the molecules of cereal β-glucan, which is initiated by coordination of the cereal β-glucan molecules to ions, such as calcium. By using low amounts of salt throughout the process, cross-linking of cereal β-glucan molecules in the intermediate solutions formed in the method of the present description, can, therefore, be minimized. In addition, by limiting the amount of salt introduced throughout the method of the present description, the cereal β-glucan can be isolated essentially free of salts in the final step of the method.

The cereal β-glucan composition prepared by the purification method of the present description generally has a purity of at least about 75%, and contains less than10% ash impurities, less than 10% protein impurities, and less than 5% lipid impurities. More particularly, the cereal β-glucan composition of the present invention has a purity of at least about 92%, and contains less than 3.5% ash impurities, less than 3.5% protein impurities, and less than 1% lipid impurities. The yield of cereal β-glucan prepared according to the purification method of the present description is generally from about 70 to about 72%.

Homogeneous solutions of the precipitated cereal β-glucan can be prepared by suspending the cereal β-glucan in reverse osmosis treated or deionized water at a temperature of about 30°C to about 45°C for a period of about 20 to about 30 minutes, or until most of the cereal β-glucan has been solubilized. The solution may then be pasteurized and a preservative added.

Aqueous solutions containing 1% cereal β-glucan, isolated according to the method of the present description, generally have the following characteristics:
- a viscosity of about 200 to about 1500 cP, more particularly about 1000 to about 1500 cP.
- a clarity value of about 5 to about 100 NTU (Nominal Turbidity Units), more particularly about 5 to about 40 NTU.
- an ash concentration of about 0.02% to about 0.2%, more particularly about 0.02% to about 0.07%.
- a protein concentration of about 0.02% to about 0.2%, more particularly about 0.02% to about 0.07%.
- a lipid concentration of about 0.005% to about 0.1 %, more particularly about 0.005% to about 0.02%.

Stabilized solutions of cereal β-glucan isolated according to the method of the present description can be prepared in the manner described in U.S. Patent No. 6,284,886. The preservatives used in the method described in U.S. Patent No. 6,284,886 should be one that is approved for human consumption and pharmaceutical use, such as, but not limited to potassium sorbate, sorbic acid, benzalkonium chloride, and parabens.

The cereal β-glucan isolated according to the method of the present description is of particular use in wound healing, and in reducing wrinkles, where transfer of cereal β-glucan across intact skin, can lead to the rebuilding of collagen through the stimulation of fibroblast growth.

The pharmaceutical composition of the present invention can be used in the form of a spray, a liquid, which may in the form of drops, or a gel. In an example, the botanical extract, and the pharmaceutically active agent comprises compounds that are readily absorbed through the mucosa of the oral cavity, the mucosa of the nasal cavity, or through gum tissue.

It is preferred that the pharmaceutical compositions of the present invention containing an anesthetic be applied to a specific, localized region of the gums or a surface of the oral cavity of a subject. It is also preferred that the compositions of the present invention, which contain a vasodilating agent, such as nitroglycerin, be applied underneath the tongue of a subject. The pharmaceutical compositions of the present invention, which comprise an antihistamine, a decongestant, a corticosteroid, or a non-steroidal anti-inflammatory drug can be applied to the back of the oral cavity, or to the nasal cavity of a subject to allow medication released from the composition to be inhaled by the subject. Pharmaceutical compositions according to the present invention, which comprise a consumable botanical extract, may be used as a mouthrinse and expectorated after being used, or, alternatively, may be swallowed.

The pharmaceutical compositions of the present invention may contain a pharmaceutically acceptable diluent or carrier, which is chosen based on the intended route of administration and standard pharmaceutical practice.

The pharmaceutical compositions of the present invention may also be administered orally in the form of tablets or capsules containing excipients, such as starch or lactose, or in the form of elixirs or suspensions containing flavoring or coloring agents. They may be injected parenterally, for example, intravenously, intramuscularly or subcutaneously. For parenteral administration, they are best used in the form of an isotonic sterile aqueous solution.

The pharmaceutical compositions of the present invention may also be administered topically when treating inflammatory conditions of the skin in the form of a cream, a jelly, a gel, a paste, or an ointment. For example, pharmaceutical compositions of the present invention, which contain, a corticosteroid, a non-steroidal anti-inflammatory drug, or a botanical extract may be used as a topical composition, in the form of a cream.

Example 2 demonstrates that β (1-3) β (1-4) glucan prepared according to the method of the present invention, and applied in the form of a topical composition to the surface of a section of skin, can significantly cross into the horny layer, the epidermis, the dermis and the subcutis layers of the skin. These results suggest that a pharmaceutically active agent or a botanical extract encapsulated by the β (1-3) β (1-4) glucan isolated according to the method of the present invention could also be effectively transferred down to the dermis and subcutis layers of the skin of a subject.

The cereal β-glucan content can be determined using a number of methods, known to those skilled in the art (McCleary AOAC method). For example, cereal β-glucan content can be assessed colorimetrically and/or by standard analytical techniques such as size exclusion chromatography and HPLC (see Wood et al. Cereal Chem. (1977) 54:524; Wood et al. Cereal Chem. (1991) 68:31-39; and Wood et al. Cereal Chem. (1991) 68:530-536). β-glucans can also be analyzed enzymatically using commercially available kits, such as Megazyme (Ireland) employing the techniques of McCleary and Glennie-Holmes J. Inst. Brew. (1985) 91:285.

Viscosities can be measured with a rotational, shear-type viscometer such as the Brookfield Syncro-Lectric or the Haake Rotovisco. Methods of using the instrument are known to those skilled in the art. Routinely, measurements are made at four speeds of disc rotation at a constant temperature of 25°C.

The following examples are provided to exemplify the present invention. Variations and alterations will be readily apparent to those skilled in the art.

### Example 1: Method for purifying cereal β-glucan derived from oat bran

Oat bran (The Quaker Oats Company) was slurried with alkaline reverse osmosis (RO) water at a pH of about 9.5 to a final solids concentration of 4-10%. The temperature was maintained at 45°C ± 5°C. The cereal β-glucan was extracted from the oat bran over a period of 30 minutes. After this time, the solids were removed by centrifugation with a decanter centrifuge. The centrate was cooled to room temperature, and the cationic flocculant SURFLOC^{®} 34030 (Jes-Chem Ltd.) was added at a 0.2% concentration. Following an incubation period of 20 minutes, coagulated particulate material was removed by centrifugation using a disk-stack centrifuge. The pH of the centrate was adjusted to approximately neutral, heated to >72°C to gelatinize starch, and treated with the heat-stable amylase Termamyl^{®} LC (Novozymes A/S). When the solution no longer produced a positive iodine test, the pH was reduced to about 4.0 to inactivate the enzyme, and the mixture was heated to 85°C for 30 minutes to denature the protein present. The solution was cooled to 4°C for one hour, and then heated to a temperature of about 72°C. An equivalent weight of CELITE^{®} C300 (World Minerals) was added to the solution, and the mixture was then filtered using a filter-press containing 25 µm filter-papers and pre-coated to a depth of about 4 mm with CELITE^{®} C65 (World Minerals). The filter press was preheated to a temperature of about 65°C, and the pH of the feedstream for the filter press was adjusted to 4.5 before the β-glucan solution was filtered. After the β-glucan solution was passed through the filter, the press was flushed with reverse osmosis water resulting in a clear, pale yellow coloured β-glucan solution. The β-glucan solution was cooled to 5°C and 95% ethanol at a temperature of -20°C was added to a final volume of about 15% (w/w) with stirring. A suspension of β-glucan was formed that was immediately separated from the solution by centrifugation with a disk-stack centrifuge. The isolated solid β-glucan was added to RO water at 41°C, allowed to disperse and then heated to between 60-70°C to produce a clear colorless solution containing about 1% β-glucan. The separated β-glucan was colourless, had a purity of greater than 75%, a viscosity >500 cP, and an exception clarity <50 NTU, as measured using a turbidity meter.

### Example 2. Quantification of the Distribution of Purified β-Glucan Applied as an Aqueous Composition to Abdominal Skin Sections

Human abdominal skin was received under informed consent from five healthy donors having undergone plastic surgery. The skin from each patient was liberated from subcutaneous fat, and cut into three sections. The skin sections were frozen in liquid nitrogen and sterilized overnight with a dose of 25 kGy of gamma-radiation. The irradiated samples were each mounted in a 20 mL volume FRANZ-CELL^{®}-like perfusion chamber (PHACOCELL^{®}, PhaCos GmbH, D-82131-Gauting, Germany; see Artmann, C. W. In vitro percutaneous absorption into human skin, Fundam. Appl. Toxicol., 28, 1-5 (1996)) containing an acceptor medium. Using a microdose applicator, the irradiated samples of skin were coated with a 5 mg/cm² dosage of Composition 1455, Composition 1450 or a control composition. The Compositions 1455 and 1450 were aqueous compositions containing 5% and 50%, respectively, of the β (1-3) β (1-4) glucan prepared according to the isolation method of the present description (see Example 1). The control composition was an aqueous composition that did not contain any β (1-3) β (1-4) glucan. The chamber was kept free of air bubbles while filling in order to ensure complete and even rinsing of the skin tissue. Pressure compensation, inside and outside of the chamber and a constant humidity of air was provided by ventilation. The skin temperature was monitored with temperature sensors, and the moisture content of the skin sections was monitored with a comeometer. The medium was regulated at 36°C and circulated continuously. Skin humidity was kept at about 65 corneometer units, and the skin surface temperature was kept at 32°C via a ventilation channel. The above conditions were maintained by regulation of the temperature of the medium by using a heating plate at the base of the chamber, and air tubes, and by adjusting the flow of air in the chamber. The skin sections were supplied by the uniformly circulating nutrient medium, which rinsed their lower surfaces. The area of application for all samples was fixed at 10 cm². The skin samples were incubated for eight hours under non-occlusive (open) conditions.

At the end of the incubation period, swab samples of the skin sections were taken with both dry cotton gauze swabs and cotton gauze swabs moistened with 0.2 mL of 70% methanol/H₂O. The skin sections were removed from the Phacocell^{®} chamber and immediately frozen in liquid nitrogen. The skin sections were then cut into 15 µm slices from the horny layer to the deeper dermis. The skin sections were allowed to air dry on clean glass slides and not fixed with any fluid. The slices were then stained with BACTIDROP^{™} Calcofluor White for 30 seconds and then washed of excess stain with deionized water. The staining and washing steps were repeated twice. The stained sample was covered with a clean glass cover slip and examined by fluorescence with a LEIKA^{®} fluorescent microscope having an exciter filter ranging between 400-500 nm with a peak of 440 nm, a barrier filter of 500-520 nm, and a xenon arc (burner) lamp. BACTIDROP^{™} Calcofluor White is a non-specific fluorochrome that binds to cellulose, and upon excitation with long wavelength ultraviolet light delineates the cell walls of cellulose-containing organisms. The deposition of the β-glucan molecules was monitored and quantified using bright fluorescence, focus inverted to white spots (3 - 5 µm) seen upon the cell walls of the samples and in the intercellular interstices.

The mean percent depositions as determined by the above fluorescence staining method are shown in Table 3. Significant fluorescent staining values (>5%) were observed in the horny layer and in the epidermis of the skin samples treated with Composition 1455 and Composition 1450. Relatively lower values were observed in the dermis and subcutis layers of the skin samples treated with Composition 1450 and Composition 1455. Fluorescence staining values of <1% were observed with the skin sections that were treated with the control composition.

**Table 3. Mean Percent Deposition of β (1-3) β (1-4) Glucan in Different Layers of Abdominal Skin**

| | Mean Percent Deposition | | | | | |
|---|---|---|---|---|---|---|
| | COMPOSITION 1455 | | COMPOSITION 1450 | | Control | |
| | Percent | Standard Deviation | Percent | Standard Deviation | Percent | Standard Deviation |
| Medium | - | - | - | - | - | - |
| Swab | - | - | - | - | - | - |
| Homy layer | 8.7 | 1.2 | 12.8 | 1.9 | 0.6 | 0.2 |
| Epidermis | 5.9 | 1.3 | 11.6 | 2.0 | 0.8 | 0.2 |
| Dermis | 2.4 | 0.5 | 4.1 | 1.1 | 0.6 | 0.1 |
| subcutis | 1.4 | 0.5 | 1.5 | 0.4 | 0.9 | 0.1 |

The documentation of the findings by photographs (not shown) also demonstrated a significant uptake of the β-glucan into the epidermis layer of the skin samples.

The measurement of fluorescence was performed in accordance with quality control procedures and documentations. Control numbers of the BACTIDROP^{™} Calcofluor White were tested using recognized quality control organisms and were found to be acceptable.(Microbiology M. Pettenkofer Institute, München). Statistical evaluation was carried out by the statistics software package SAS/STATISTICA^{®}. Both the hardware and the software used were validated.

### Example 3. Preparation of a mouthwash or spray containing an avenanthramide-containing extract.

1 g of an oat extract containing 100 pm avenanthramide (Ceapro Inc.) was added to a stirred 10 % (w/w) solution of oat β (1-3) β (1-4) glucan (Ceapro Inc.) producing a clear near-colourless solution.

### Example 4. Preparation of a mouthwash or spray containing a nutraceutical extract

An extract of *Echinacea angustifolia* (1000 mg) in 45% ethanol was added to a stirred solution of oat beta glucan (Ceapro Inc.) to achieve a final concentration of 0.5 % w/w oat beta glucan. The mixture was evaporated under reduced vacuum to remove the alcohol, affording a clear light amber solution.

The present invention has been described with regard to preferred embodiments. However, it will be obvious to persons skilled in the art that a number of variations and modifications can be made.

## Claims

1. A pharmaceutical composition comprising:
a β (1-3) β (1-4) glucan, and
a botanical extract, or a pharmaceutically active agent,
wherein the β (1-3) β (1-4) glucan is produced by a method comprising:
(i) extracting a milled cereal grain or a milled part of the cereal grain with an alkaline solution to produce an extract containing at least about 0.4 weight percent β (1-3) β (1-4) glucan;
(ii) removing insoluble material, and removing particulate material having a particle size of greater than about 0.2 µm from said extract to produce a purified extract;
(iii) adding from about 10% to about 25% (vol/vol) of a C₁-C₄ alcohol to the purified extract to precipitate the β (1-3) β (1-4) glucan, and
(iv) isolating the β(1-3) β(1-4) glucan.

2. The pharmaceutical composition according to claim 1, wherein the composition comprises the botanical extract, and wherein the botanical extract is an extract of Guarana, *Ginkgo biloba,* Kola nut, Goldenseal, Golo Kola, *Schizandra,* Elderberry, St. John's Wort, Valerian and *Ephedra*, black tea, white tea, java tea, garlic oil, fiber, green tea, lemon oil, mace, licorice, onion oil, orange oil, rosemary, milk thistle, *Echinacea,* Siberian ginseng or *Panax ginseng,* lemon balm, *Kava kava*, matte, bilberry, soy, grapefruit, seaweed, hawthorn, lime blossom, sage, clove, basil, curcumin, taurine, wild oat herb, oat grain, dandelion, gentian, aloe vera, hops, cinnamon, peppermint, grape, chamomile, fennel, marshmallow, ginger, slippery elm, cardamon, coriander, anise, thyme, rehmannia, eucalyptus, menthol, schisandra, withania, cowslip, lycium, or passion flower.

3. The pharmaceutical composition according to claim 2, wherein the botanical extract is an extract of oat grain.

4. The pharmaceutical composition of claim 3, wherein the botanical extract comprises avenanthramide.

5. The pharmaceutical composition according to claim 1, wherein the composition comprises the pharmaceutically active agent, and wherein the pharmaceutically active agent is selected from the group consisting of beta-sitosterol, caffeine, cafestol, D-limonene, kabweol, nomilin, oltipraz, sulphoraphane, tangeretin, folic acid, and menthol.

6. The pharmaceutical composition according to claim 1, wherein the composition comprises the pharmaceutically active agent, and wherein the pharmaceutically active agent is selected from the group consisting of an antihistamine, a decongestant, a corticosteroid, a non-steroidal anti-inflammatory drug, a bronchodilator, a vasodilator, such as nitroglycerin, and a local anaesthetic.

7. The pharmaceutical composition of claim 6, wherein the vasodilator is nitroglycerin.

8. The pharmaceutical composition according to claim 1, wherein the β (1-3) β (1-4) glucan is derived from a cereal grain or a part of the cereal grain.

9. The pharmaceutical composition according to claim 8, wherein the cereal is selected from the group consisting of a cultivar of barley, a cultivar of oat, a cultivar of wheat, a cultivar of rye, a cultivar of sorghum, a cultivar of millet, a cultivar of corn, and a mixture thereof.

10. The pharmaceutical composition according to claim 1, wherein the β (1-3) β (1-4) glucan is a β (1-3) β (1-4) glucan composition having a purity of at least about 75%, and containing less than 10% ash impurities, less than 10% protein impurities, and less than 5% lipid impurities.

11. The pharmaceutical composition according to claim 10, wherein the β (1-3) β (1-4) glucan composition has a purity of at least about 92%, and contains less than 3.5% ash impurities, less than 3.5 % protein impurities, and less than 1% lipid impurities.

12. The pharmaceutical composition according to claim 10, wherein the cereal β-glucan, composition has a clarity value of from about 5 to about 100 NTU.

13. The pharmaceutical composition according to claim 1, wherein in said step of adding (step iii) in said method, about 10% to about 20% (vol/vol) of an alcohol selected from the group consisting of methanol, ethanol and isopropanol, is used to precipitate the β (1-3) β (1-4) glucan composition from said purified extract.

14. The pharmaceutical composition according to claim 13, wherein about 10% to about 20% (vol/vol) of ethanol is used to precipitate the β (1-3) β (1-4) glucan composition from said purified extract.

15. The pharmaceutical composition according to claim 1, wherein said step of removing particulate material in said method comprises:
one, or more than one step of adding a flocculant, a coagulant or both the flocculant and
the coagulant to said extract to coagulate particulate material having a particle size of greater than about 0.2 µm, and removing coagulated material from said extract,
digesting starch material in said extract, and
filtering out particulate material having a particle size of greater than about 0.2 µm from said extract to produce the purified extract.

16. The pharmaceutical composition according to claim 15, wherein in said step of digesting in said method, said starch material is digested with an enzyme.

17. The pharmaceutical composition according to claim 16, wherein prior to digesting said starch material, said alkaline solution is neutralized.

18. The pharmaceutical composition according to claim 17, wherein following the digestion of said starch material in said method, said enzyme is inactivated.

19. The pharmaceutical composition according to claim 18, wherein said enzyme is inactivated by acidifying the neutralized solution.

20. The pharmaceutical composition according to claim 16, wherein said enzyme is an amylase.

21. The pharmaceutical composition according to claim 20, wherein said amylase does not require a calcium cofactor.

22. The pharmaceutical composition according to claim 1, wherein the cereal is selected from the group consisting of a cultivar of barley, a cultivar of oat, a cultivar of wheat, a cultivar of rye, a cultivar of sorghum, a cultivar of millet, a cultivar of corn, and a mixture thereof.

23. The pharmaceutical composition according to claim 1, wherein the alkaline solution has a value of pH of between 9 to 10.

24. The pharmaceutical composition according to claim 1, wherein the pH of the alkaline solution used in said method is from about 9.25 to about 9.75.

25. The pharmaceutical composition according to claim 1, wherein said step of adding (step iii) in said method is conducted at a temperature of from about 1°C to about 10°C.

26. The pharmaceutical composition according to claim 1, wherein said method further comprises one, or more than one step of dissolving the isolated β (1-3) β (1-4) glucan in an aqueous solution, precipitating the β (1-3) β (1-4) glucan by adding about 10% to about 25% (vol/vol) of the C₁-C₄ alcohol to the aqueous solution, and isolating the β (1-3) β (1-4) glucan.

27. The pharmaceutical composition according to claim 23 or 24, wherein the milled cereal grain or the milled part of the cereal grain is extracted with the alkaline solution for a period of time of about 15 to about 45 minutes to produce the extract containing at least about 0.4 weight percent β (1-3) β (1-4) glucan.

28. The pharmaceutical composition according to claim 15, wherein the step of filtering out material having a particle size of greater than 0.2 µm from said extract is conducted using a filter coated with a pre-coat of a filter aid having a porosity of 0.2 µm.

## Patentansprüche

1. Pharmazeutische Zusammensetzung, umfassend:
ein β(1-3)-β(1-4)-Glucan und
einen botanischen Extrakt oder einen pharmazeutischen Wirkstoff,
wobei das β(1-3)-β(1-4)-Glucan durch ein Verfahren hergestellt wird, bei dem man:
(i) ein gemahlenes Getreidekorn oder einen gemahlenen Teil des Getreidekorns mit einer alkalischen Lösung extrahiert, wodurch man einen Extrakt erhält, der mindestens etwa 0,4 Gewichtsprozent β(1-3)-β(1-4)-Glucan enthält;
(ii) unlösliches Material entfernt und teilchenförmiges Material mit einer Teilchengröße von mehr als etwa 0,2 µm aus dem Extrakt entfernt, wodurch man einen aufgereinigten Extrakt erhält;
(iii) etwa 10 Vol.-% bis etwa 25 Vol.-% eines C₁-C₄-Alkohls zu dem aufgereinigten Extrakt gibt, um das β(1-3)-β(1-4)-Glucan auszufällen, und
(iv) das β(1-3)-β(1-4)-Glucan isoliert.

2. Pharmazeutische Zusammensetzung nach Anspruch 1, wobei die Zusammensetzung den botanischen Extrakt umfasst, und wobei es sich bei dem botanischen Extrakt um einen Extrakt von Guaraná, *Ginkgo biloba*, Kolanuss, kanadischer Orangenwurzel (Goldenseal), Golo Kola, *Schizandra*, Holunderbeere, Johanniskraut, Baldrian und Meerträubel (*Ephedra*), schwarzem Tee, weißem Tee, Java-Tee, Knoblauchöl, Faser, grünem Tee, Zitronenöl, Macis, Lakritze, Zwiebelöl, Orangenöl, Rosmarin, Milchdistel, *Echinacea*, siberischem Ginseng oder *Panax ginseng,* Zitronenmelisse, *Kava kava*, Mate, Heidelbeere, Soja, Grapefruit, Seetang, Weißdorn, Lindenblüte, Salbei, Nelke, Basilikum, Curcumin, Taurin, Wildhaferkraut, Haferkorn, Löwenzahn, Enzian, Aloe vera, Hopfen, Zimt, Pfefferminze, Weintraube, Kamille, Fenchel, echtem Eibisch, Ingwer, Rot-Ulme, Kardamon, Koriander, Anis, Thymian, *Rehmannia*, Eukalyptus, Menthol, Spaltkörbchen (*Schisandra*), *Withania*, Schlüsselblume, *Lycium* oder Passionsblume handelt.

3. Pharmazeutische Zusammensetzung nach Anspruch 2, wobei es sich bei dem botanischen Extrakt um einen Extrakt von Haferkorn handelt.

4. Pharmazeutische Zusammensetzung nach Anspruch 3, wobei der botanische Extrakt Avenanthramid umfasst.

5. Pharmazeutische Zusammensetzung nach Anspruch 1, wobei die Zusammensetzung den pharmazeutischen Wirkstoff umfasst, und wobei der pharmazeutische Wirkstoff aus der aus beta-Sitosterol, Coffein, Cafestol, D-Limonen, Kahweol, Nomilin, Oltipraz, Sulforaphan, Tangeritin, Folsäure und Menthol bestehenden Gruppe ausgewählt ist.

6. Pharmazeutische Zusammensetzung nach Anspruch 1, wobei die Zusammensetzung den pharmazeutischen Wirkstoff umfasst, und wobei der pharmazeutische Wirkstoff aus der aus einem Antihistamin, einem schleimhautabschwellenden Mittel, einem Kortikosteroid, einem nichtsteroidalen entzündungshemmenden Mittel, einem Bronchodilator, einem Vasodilator wie Nitroglyzerin und einem Lokalanästhetikum bestehenden Gruppe ausgewählt ist.

7. Pharmazeutische Zusammensetzung nach Anspruch 6, wobei es sich bei dem Vasodilator um Nitroglyzerin handelt.

8. Pharmazeutische Zusammensetzung nach Anspruch 1, wobei das β(1-3)-β(1-4)-Glucan aus einem Getreidekorn oder einem Teil des Getreidekorns gewonnen wurde.

9. Pharmazeutische Zusammensetzung nach Anspruch 8, wobei das Getreide aus der aus einem Gerstenkultivar, einem Haferkultivar, einem Weizenkultivar, einem Roggenkultivar, einem Sorghumkultivar, einem Hirsekultivar, einem Maiskultivar und einer Mischung davon bestehenden Gruppe ausgewählt ist.

10. Pharmazeutische Zusammensetzung nach Anspruch 1, wobei es sich bei dem β(1-3)-β(1-4)-Glucan um eine β(1-3)-β(1-4)-Glucanzusammensetzung mit einer Reinheit von mindestens etwa 75% handelt, die weniger als 10% Ascheverunreinigungen, weniger als 10% Proteinverunreinigungen und weniger als 5% Lipidverunreinigungen enthält.

11. Pharmazeutische Zusammensetzung nach Anspruch 10, wobei die β(1-3)-β(1-4)-Glucanzusammensetzung eine Reinheit von mindestens etwa 92% hat und weniger als 3,5% Ascheverunreinigungen, weniger als 3,5% Proteinverunreinigungen und weniger als 1% Lipidverunreinigungen enthält.

12. Pharmazeutische Zusammensetzung nach Anspruch 10, wobei die Getreide-β-Glucanzusammensetzung einen Klarheitsgrad von etwa 5 bis etwa 100 NTU aufweist.

13. Pharmazeutische Zusammensetzung nach Anspruch 1, wobei bei dem Schritt der Zugabe (Schritt iii) bei dem Verfahren die β(1-3)-β(1-4)-Glucanzusammensetzung aus dem aufgereinigten Extrakt mit etwa 10 Vol.-% bis etwa 20 Vol.-% eines Alkohols, ausgewählt aus der aus Methanol, Ethanol und Isopropanol bestehenden Gruppe, ausgefällt wird.

14. Pharmazeutische Zusammensetzung nach Anspruch 13, wobei die β(1-3)-β(1-4)-Glucanzusammensetzung aus dem aufgereinigten Extrakt mit etwa 10 Vol.-% bis etwa 20 Vol.-% Ethanol ausgefällt wird.

15. Pharmazeutische Zusammensetzung nach Anspruch 1, wobei der Schritt des Entfernens des teilchenförmigen Materials bei dem Verfahren Folgendes umfasst:
einen oder mehr als einen Schritt einer Zugabe eines Flockungsmittels, eines Koagulationsmittels oder sowohl des Flockungsmittels als auch des Koagulationsmittels zu dem Extrakt, um teilchenförmiges Material mit einer Teilchengröße von mehr als etwa 0,2 µm zum Koagulieren zu bringen, und Entfernen des koagulierten Materials aus dem Extrakt,
Verdauen des Stärkematerials in dem Extrakt und
Abfiltrieren von teilchenförmigem Material mit einer Teilchengröße von mehr als etwa 0,2 µm aus dem Extrakt unter Gewinnung des aufgereinigten Extrakts.

16. Pharmazeutische Zusammensetzung nach Anspruch 15, wobei man bei dem Schritt des Verdauens in dem Verfahren das Stärkematerial mit einem Enzym verdaut.

17. Pharmazeutische Zusammensetzung nach Anspruch 16, wobei man vor dem Verdauen des Stärkematerials die alkalische Lösung neutralisiert.

18. Pharmazeutische Zusammensetzung nach Anspruch 17, wobei man nach dem Verdauen des Stärkematerials in dem Verfahren das Enzym deaktiviert.

19. Pharmazeutische Zusammensetzung nach Anspruch 18, wobei man das Enzym durch Ansäuern der neutralisierten Lösung deaktiviert.

20. Pharmazeutische Zusammensetzung nach Anspruch 16, wobei es sich bei dem Enzym um eine Amylase handelt.

21. Pharmazeutische Zusammensetzung nach Anspruch 20, wobei die Amylase keinen Kalzium-Cofaktor benötigt.

22. Pharmazeutische Zusammensetzung nach Anspruch 1, wobei das Getreide aus der aus einem Gerstekultivar, einem Haferkultivar, einem Weizenkultivar, einem Roggenkultivar, einem Sorghumkultivar, einem Hirsekultivar, einem Maiskultivar und einer Mischung davon bestehenden Gruppe ausgewählt ist.

23. Pharmazeutische Zusammensetzung nach Anspruch 1, wobei die alkalische Lösung einen pH-Wert zwischen 9 und 10 aufweist.

24. Pharmazeutische Zusammensetzung nach Anspruch 1, wobei der pH-Wert der in dem Verfahren eingesetzten alkalischen Lösung sich auf etwa 9,25 bis etwa 9,75 beläuft.

25. Pharmazeutische Zusammensetzung nach Anspruch 1, wobei man den Schritt der Zugabe (Schritt iii) in dem Verfahren bei einer Temperatur von etwa 1°C bis etwa 10°C durchführt.

26. Pharmazeutische Zusammensetzung nach Anspruch 1, wobei das Verfahren weiterhin einen oder mehr als einen Schritt des Auflösens des isolierten β(1-3)-β(1-4)-Glucans in einer wässrigen Lösung, des Ausfällens des β(1-3)-β(1-4)-Glucans durch Zugabe von etwa 10 Vol.-% bis etwa 25 Vol.-% des C₁-C₄-Alkohls zu der wässrigen Lösung und des Isolierens des β(1-3)-β(1-4)-Glucans umfasst.

27. Pharmazeutische Zusammensetzung nach Anspruch 23 oder 24, wobei das gemahlene Getreidekorn oder der gemahlene Teil des Getreidekorns mit der alkalischen Lösung über eine Zeitspanne von etwa 15 bis etwa 45 Minuten extrahiert wird, wodurch man den Extrakt erhält, der mindestens etwa 0,4 Gewichtsprozent β(1-3)-β(1-4)-Glucan enthält.

28. Pharmazeutische Zusammensetzung nach Anspruch 15, wobei man den Schritt des Abfiltrierens von Material mit einer Partikelgröße von mehr als 0,2 µm aus dem Extrakt mit einem mit einer Vorbeschichtung eines Filtrierhilfsmittels mit einer Porosität von 0,2 µm beschichteten Filter durchführt.

## Revendications

1. Composition pharmaceutique comprenant :
un β (1-3) β (1-4) glucane, et
un extrait botanique, ou un agent pharmaceutiquement actif,
le β (1-3) β (1-4) glucane étant produit par un procédé comprenant :
(i) l'extraction de grain de céréale moulu ou d'une partie moulue du grain de céréale avec une solution alcaline pour produire un extrait contenant au moins environ 0,4 pour cent en poids de β (1-3) β (1-4) glucane ;
(ii) l'élimination de la matière insoluble, et l'élimination de la matière particulaire ayant une taille de particule supérieure à environ 0,2 µm dudit extrait pour produire un extrait purifié ;
(iii) l'addition d'environ 10 % à environ 25 % (vol/vol) d'un alcool en C₁-C₄ à l'extrait purifié pour précipiter le β (1-3) β (1-4) glucane, et
(iv) l'isolement du β(1-3) β(1-4) glucane.

2. Composition pharmaceutique selon la revendication 1, **caractérisée en ce que** la composition comprend l'extrait botanique, et **en ce que** l'extrait botanique est un extrait de Guarana, *Ginkgo biloba*, noix de kola, sceau d'or, Golo Kola, *Schizandra*, baie de sureau, millepertuis, valériane et *Ephedra*, thé noir, thé blanc, thé de Java, huile d'ail, fibre, thé vert, huile de citron, macis, réglisse, huile d'oignon, huile d'orange, romarin, chardon-marie, *Echinacea*, ginseng de Sibérie ou *Panax ginseng,* citronnelle, *Kava kava*, matte, myrtille, soja, pamplemousse, algue, aubépine, fleur de citron vert, sauge, clou de girofle, basilique, curcumine, taurine, herbe d'avoine sauvage, graine d'avoine, dent de lion, gentiane, aloe vera, houblon, cannelle, menthe poivrée, raisin, camomille, fenouil, guimauve, gingembre, orme rouge, cardamome, coriandre, anis, thym, rehmannia, eucalyptus, menthol, schisandra, withania, primevère, lycium ou passiflore.

3. Composition pharmaceutique selon la revendication 2, **caractérisée en ce que** l'extrait botanique est un extrait de graine d'avoine.

4. Composition pharmaceutique selon la revendication 3, **caractérisée en ce que** l'extrait botanique comprend l'avenanthramide.

5. Composition pharmaceutique selon la revendication 1, **caractérisée en ce que** la composition comprend l'agent pharmaceutiquement actif, et **en ce que** l'agent pharmaceutiquement actif est choisi dans le groupe constitué par le bêta-sitostérol, la caféine, le cafestol, le D-limonène, le kahweol, la nomiline, l'oltipraz, le sulforaphane, la tangérétine, l'acide folique et le menthol.

6. Composition pharmaceutique selon la revendication 1, **caractérisée en ce que** la composition comprend l'agent pharmaceutiquement actif, et **en ce que** l'agent pharmaceutiquement actif est choisi dans le groupe constitué par un antihistaminique, un décongestionnant, un corticostéroïde, un anti-inflammatoire non stéroïdien, un bronchodilatateur, un vasodilatateur, tel que la nitroglycérine, et un anesthésique local.

7. Composition pharmaceutique selon la revendication 6, **caractérisée en ce que** le vasodilatateur est la nitroglycérine.

8. Composition pharmaceutique selon la revendication 1, **caractérisée en ce que** le β (1-3) β (1-4) glucane est issu d'un grain de céréale ou d'une partie du grain de céréale.

9. Composition pharmaceutique selon la revendication 8, **caractérisée en ce que** la céréale est choisie dans le groupe constitué par un cultivar d'orge, un cultivar d'avoine, un cultivar de blé, un cultivar de seigle, un cultivar de sorgho, un cultivar de millet, un cultivar de maïs, et un mélange de ceux-ci.

10. Composition pharmaceutique selon la revendication 1, **caractérisée en ce que** le β (1-3) β (1-4) glucane est une composition de β (1-3) β (1-4) glucane ayant une pureté d'au moins environ 75 %, et contenant moins de 10 % d'impuretés de cendre, moins de 10 % d'impuretés de protéine, et moins de 5 % d'impuretés de lipide.

11. Composition pharmaceutique selon la revendication 10, **caractérisée en ce que** la composition de β (1-3) β (1-4) glucane a une pureté d'au moins environ 92 %, et contient moins de 3,5 % d'impuretés de cendre, moins de 3,5 % d'impuretés de protéine, et moins de 1 % d'impuretés de lipide.

12. Composition pharmaceutique selon la revendication 10, **caractérisée en ce que** la composition de β-glucane de céréale a un indice de clarté d'environ 5 à environ 100 NTU.

13. Composition pharmaceutique selon la revendication 1, **caractérisée en ce que** dans ladite étape d'addition (étape iii) dans ledit procédé, on utilise d'environ 10 % à environ 20 % (vol/vol) d'un alcool choisi dans le groupe constitué par le méthanol, l'éthanol et l'isopropanol pour précipiter la composition de β (1-3) β (1-4) glucane dans ledit extrait purifié.

14. Composition pharmaceutique selon la revendication 13, **caractérisée en ce qu'**on utilise d'environ 10 % à environ 20 % (vol/vol) d'éthanol pour précipiter la composition de β (1-3) β (1-4) glucane dans ledit extrait purifié.

15. Composition pharmaceutique selon la revendication 1, **caractérisée en ce que** ladite étape d'élimination de matière particulaire dans ledit procédé comprend :
une ou plusieurs étapes d'addition d'un floculant, d'un coagulant ou à la fois d'un floculant et d'un coagulant audit extrait pour coaguler la matière particulaire ayant une taille de particule supérieure à environ 0,2 µm, et d'élimination de la matière coagulée dudit extrait,
la digestion de la matière amylacée dans ledit extrait, et
la filtration de la matière particulaire ayant une taille de particule supérieure à environ 0,2 µm dudit extrait pour produire l'extrait purifié.

16. Composition pharmaceutique selon la revendication 15, **caractérisée en ce que** dans ladite étape de digestion dans ledit procédé, ladite matière amylacée est digérée par une enzyme.

17. Composition pharmaceutique selon la revendication 16, **caractérisée en ce qu'**avant la digestion de ladite matière amylacée, ladite solution alcaline est neutralisée.

18. Composition pharmaceutique selon la revendication 17, **caractérisée en ce qu'**après la digestion de ladite matière amylacée dans ledit procédé, ladite enzyme est inactivée.

19. Composition pharmaceutique selon la revendication 18, **caractérisée en ce que** ladite enzyme est inactivée par acidification de la solution neutralisée.

20. Composition pharmaceutique selon la revendication 16, **caractérisée en ce que** ladite enzyme est une amylase.

21. Composition pharmaceutique selon la revendication 20, **caractérisée en ce que** ladite amylase ne nécessite pas de cofacteur calcium.

22. Composition pharmaceutique selon la revendication 1, **caractérisée en ce que** la céréale est choisie dans le groupe constitué par un cultivar d'orge, un cultivar d'avoine, un cultivar de blé, un cultivar de seigle, un cultivar de sorgho, un cultivar de millet, un cultivar de maïs, et un mélange de ceux-ci.

23. Composition pharmaceutique selon la revendication 1, **caractérisée en ce que** la solution alcaline a une valeur de pH comprise entre 9 et 10.

24. Composition pharmaceutique selon la revendication 1, **caractérisée en ce que** le pH de la solution alcaline utilisée dans ledit procédé est d'environ 9,25 à environ 9,75.

25. Composition pharmaceutique selon la revendication 1, **caractérisée en ce que** ladite étape d'addition (étape iii) dans ledit procédé est effectuée à une température d'environ 1°C à environ 10°C.

26. Composition pharmaceutique selon la revendication 1, **caractérisée en ce que** ledit procédé comprend en outre une ou plusieurs étapes de dissolution du β (1-3) β (1-4) glucane isolé dans une solution aqueuse, de précipitation du β (1-3) β (1-4) glucane en ajoutant environ 10 % à environ 25 % (vol/vol) de l'alcool en C₁-C₄ à la solution aqueuse, et d'isolement du β (1-3) β (1-4) glucane.

27. Composition pharmaceutique selon la revendication 23 ou 24, **caractérisée en ce que** le grain de céréale moulu ou la partie moulue du grain de céréale est extrait avec la solution alcaline pendant une durée de temps d'environ 15 à environ 45 minutes pour produire un extrait contenant au moins environ 0,4 pour cent en poids de β (1-3) β (1-4) glucane.

28. Composition pharmaceutique selon la revendication 15, **caractérisée en ce que** l'étape de filtration de matière ayant une taille de particule supérieure à 0,2 µm dudit extrait est effectuée en utilisant un filtre revêtu d'une pré-couche d'adjuvant de filtration ayant une porosité de 0,2 µm.
